# EUROPÄISCHE PATENTANMELDUNG

(11) **EP 0 752 283 A1**
(43) Veröffentlichungstag der Anmeldung: **08.01.1997**
(21) Anmeldenummer: 96106065.4
(22) Anmeldetag: 18.04.1996
(51) Int. Cl.: B08B 9/46, G01N 1/26, G01N 33/44

(54) **Verfahren und Vorrichtung zur Prüfung von Behältern**

(30) Priorität: 05.07.1995 CH 1964/95
(71) Anmelder: ELPATRONIC AG, 6303 Zug (CH)
(72) Erfinder: Gernet, Pierre, 5400 Ennetbaden (CH)

(57) **Zusammenfassung**

Bei der Prüfung von Behältern (2), welche auf einer Förderanlage (1) gefördert werden, wird den Behältern durch einen Schnüffler (3) Gas entnommen, welches einer Analyse zugeführt wird. Um die Gasentnahme zu erleichtern, wird vor der Gasentnahmeposition in die Behälter mittels mindestens einer Düse (5) Druckluft eingeblasen, wobei der Abstand zwischen Düse und Gasentnahmeposition so gewählt wird, dass eine optimale Analyse des entnommenen Gases möglich wird.

## Beschreibung

Die Erfindung betrifft ein Verfahren zur Prüfung von Behältern, insbesonders von Mehrweg-Flaschen, auf das Vorhandensein von Verunreinigungen, bei welchem durch eine Prüfeinrichtung in die auf einer Förderanlage geförderten Behälter ein Gas oder Gasgemisch, insbesondere Luft eingeblasen und aus den Behältern Gasproben entnommen und analysiert werden. Ferner betrifft die Erfindung eine Vorrichtung zur Prüfung von Behältern, insbesonders Mehrweg-Flaschen, mit einer Prüfeinrichtung für die auf einer Förderanlage geförderten Behälter, wobei die Prüfeinrichtung zur Einblasung eines Gases oder Gasgemisches, insbesondere Luft, in die Behälter und zur Entnahme einer Gasprobe aus den Behältern ausgestaltet ist.

Ein entsprechendes Verfahren bzw. eine solche Vorrichtung ist aus EP-A-0 579 952 bekannt. Dort werden aus den Behältern mittels Sonden Gasproben entnommen und analysiert, um Verunreinigungen der Flaschen zu erkennen. In den Sonden sind Leitungen zur Einblasung von Luft während der Probeentnahme vorgesehen, um die Konzentration der in der Gasprobe enthaltenen Verunreinigungen zu erhöhen, indem die eingeblasene Luft die in dem Behälter befindliche Luft, die analysierbare Spuren der Verunreinigungen enthält, aus dem Behälter verdrängt und dem Probeentnahmeorgan zuführt. Das in EP-A-0 579 952 geschilderte Konzept mit beisammenliegenden Einblasmitteln und Probeentnahmemitteln, die zusammen in den Behälter eintauchen, hat sich bewährt, wenn genügend Zeit zur Lufteinblasung und Probeentnahme zur Verfügung steht. Dies ist bei der Prüfung von rücklaufenden Mehrweg-Flaschen auch bei hohen Flaschendurchlaufraten z.B. dann der Fall, wenn eine Prüfvorrichtung mit einem Karussell verwendet wird, durch welches auch bei hohen Durchlaufraten eine genügende Verweilzeit der Flaschen in der Prüfvorrichtung sichergestellt wird. Eine Prüfvorrichtung mit Karussell ist aber technisch aufwendig und entsprechend kostspielig. Es stellt sich deshalb das Problem auch bei solchen Förderanlagen, bei denen die Verweilzeit der Behälter kürzer ist, insbesondere linearen Förderanlagen, eine sichere Gasprobenentnahme bzw. Analyse zu ermöglichen. Insbesondere stellt sich das Problem auch bei der Prüfung von Glasflaschen, deren Förderlinien mit sehr hohen Geschwindigkeiten (z.B. mit 40'000 bis 60'000 Flaschen pro Stunde) arbeiten und bei welchen solche Verunreinigungen wie Lösungsmittel, Benzin, Petrol, Diesel und Motorenöl sicher erkannt werden sollen, da diese Stoffe schon in geringen Mengen die Waschlauge, mit der die Flaschen gewaschen werden, unbrauchbar machen können.

Die Aufgabe wird bei einem Verfahren der eingangs genannten Art dadurch gelöst, dass die Gaseinblasung erfolgt, bevor der jeweilige Behälter die Gasprobenentnahmeposition erreicht.

Anstelle der bisherigen Einblasung des Gases an der Gasentnahmeposition wird erfindungsgemäss das Einblasen durchgeführt bevor der Behälter die Gasentnahmeposition erreicht hat. Dieses Einblasen mit Vorhalt bzw. mit einem räumlichen und zeitlichen Abstand von der Gasentnahmeposition löst die Aufgabe. Wenn der Behälter die Gasentnahmeposition erreicht hat, ist die Einblasung bereits abgeschlossen und das eingeblasene Gas hat die im Behälter befindliche Luft bereits beeinflussen können, so dass diese Luft zur Entnahme bereit ist. Die gesamte Verweilzeit des Behälters bei der Gasentnahmeposition steht somit zur Gasentnahme zur Verfügung und es muss in dieser Position nicht noch auf die Einblasung und/oder das Wirksamwerden des eingeblasenen Gases gewartet werden.

Bei einer Vorrichtung der eingangs genannten Art wird die Aufgabe dadurch gelöst, dass mindestens ein Einblasmittel in Förderrichtung vor der Gasprobenentnahmestelle angeordnet ist, durch welches Gas in den Behälter einblasbar ist, bevor der Behälter die Gasentnahmeposition erreicht.

Bevorzugterweise erfolgt die Gaseinblasung in einem vorbestimmten und einstellbaren Abstand von der Gasentnahmeposition. Dazu können bei der Vorrichtung mehrere Düsen vorgesehen sein. Vorzugsweise wird weiter der Abstand in Abhängigkeit von der Behälterhöhe und/oder der Fördergeschwindigkeit eingestellt. Weiter bevorzugt ist auch eine Regelung des Abstandes z.B. in Abhängigkeit von der Fördergeschwindigkeit.

Im folgenden werden Ausführungsbeispiele der Erfindung anhand der Figuren näher erläutert. Dabei zeigt
Figur 1 grob schematisch eine Förderanlage mit einer Prüfvorrichtung; und
Figur 2 eine weitere Ausführungsform der Vorrichtung ebenfalls stark schematisierter Darstellung.

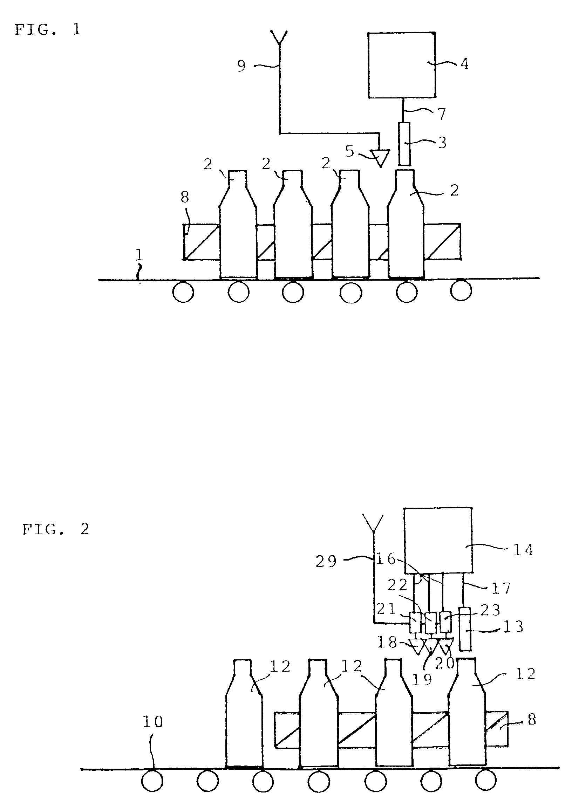

Figur 1 zeigt einen Abschnitt 1 einer Förderanlage für die Behälter 2. Dabei kann es sich, wie in der Figur angedeutet, um eine lineare Förderanlage handeln. Es kann aber auch eine Förderanlage mit einer gekrümmten Bahn oder insbesondere auch eine Karussellförderanlage sein. Im gezeigten Beispiel der linearen Förderanlage ist zusätzlich ein Schneckenförderer 8 vorgesehen, welcher eine genau definierte Position der Behälter im Bereich der Prüfeinrichtung bewirkt. Die Flaschen können andererseits auch in nicht genau gleichmässigen Abständen ungeführt gefördert werden (ohne den Schneckenförderer). Durch eine Erfassung der Position der jeweiligen Flasche wird dann der Einblasvorgang und eventuell der Schnüffelvorgang durch die Flasche selber ausgelöst.

Die Prüfeinrichtung umfasst einerseits einen Schnüffler, mittels welchem Gasproben aus dem Behälter entnommen und einer Analyse zugeführt werden. In der Figur 1 ist der Schnüffler durch eine Sonde 3 angedeutet, welche oberhalb der Behältermündung des jeweiligen Behälters 2 Gas absaugt und über eine Leitung 7 der Analyseeinrichtung 4 zuführt. Natürlich ist diese schematisch dargestellte Ausführung der Gasprobenentnahme nur als Beispiel zu verstehen. Es könnte auch eine Gasprobenentnahme gemäss der EP-A-0 579 952, vorgesehen sein, also eine Gasprobenentnahme mit einem in den jeweiligen Behälter eintauchenden Gasprobenentnahmerohr. Auch eine beliebige andere Ausgestaltung der Gasprobenentnahme mit einem in den jeweiligen Behälter eintauchenden Element oder einem nur über dem Behälter angeordneten Element kann vorgesehen sein. Die aus dem Behälter entnommene Gasprobe wird der Analyseeinrichtung 4 zugeführt. Dabei handelt es sich um bekannte Analyseeinrichtungen, wie z.B. ein Massenspektrometer, einen Photoionisationsdetektor oder einen Sensor für polyzyklische aromatische Kohlenwasserstoffe. Diese Sensoren sind zur Prüfung von Gasproben aus Behältern bereits bekannt und handelsüblich und werden daher hier nicht näher beschrieben.

Bei einer schnellaufenden Förderanlage ergibt sich nur eine sehr kurze Verweilzeit des jeweiligen Behälters bei der Gasentnahmeposition beim Schnüffler 3. Bei z.B. 1000 Flaschen/Minute und 100 mm Flaschenabstand werden pro Sekunde 16,7 Flaschen mit einer Geschwindigkeit von 1700 mm/Sekunde gefördert. Bei einem Halsdurchmesser von 18 mm der Flasche liegt die Oeffnung nur gerade 10,5 ms vor dem Schnüffler 3. Gemäss der Erfindung erfolgt nun ein Einblasen von Luft in den jeweiligen Behälter, bevor dieser die Gasentnahmeposition beim Schnüffler 3 erreicht. In der Figur 1 ist dazu eine Düse 5 oberhalb der Behälter 2 vorgesehen, mittels welcher ein Gas, vorzugsweise Luft, in den jeweiligen Behälter einblasbar ist, wenn dieser die Düse passiert und bevor dieser die Gasentnahmeposition beim Schnüffler 3 erreicht. Die Düse 5 wird durch eine Leitung 9 aus einer nicht dargestellten Druckluftquelle mit Druckluft gespeist. Die Einblasung von Luft kann dabei z.B. mit einer Luftgeschwindigkeit von 50 m/Sek. erfolgen. Bei einer Flaschenhöhe von 300 mm ergibt sich dann eine Laufzeit der eingeblasenen Luft bis zum Ausstossen der in der Flasche liegenden Luft von ungefähr 6-10 ms. Dies entspricht bei der angenommenen Flaschengeschwindigkeit, einem räumlichen Abstand von ungefähr 10 mm bis 17 mm von der Düse 5 bis zur Gasentnahmeposition beim Schnüffler 3. Der genannte Abstand zwischen der Einblasdüse 5 und dem Schnüffler 3 richtet sich also nach der Zeitkonstante Einblas/Ausstoss-Reaktionszeit und Geschwindigkeit der Förderanlage. Es ist daher bevorzugt, wenn die Düse 5 in ihrem Abstand von der Gasentnahmeposition einstellbar ist, damit eine Anpassung des Abstandes an die genannten Parameter möglich ist. Es ist auch möglich, dass die Düse durch einen nicht dargestellten Antrieb in ihrem Abstand vom Schnüffler einstellbar ist. Der Abstand kann dann durch eine Regelung immer so eingestellt werden, in Abhängigkeit von der Fördergeschwindigkeit, dass für die Gasentnahme eine immer gleichbleibende optimale Situation erzielt wird. Anstelle der Düse kann natürlich auch die Lage des Schnüfflers geändert werden, um den Abstand einzustellen oder zu regeln.

Figur 2 zeigt eine Ausführungsform, bei welcher mehrere Düsen vorgesehen sind. Damit ist der Abstand durch Auswahl der entsprechenden Düse einstellbar. Im einzelnen zeigt Figur 2 eine Förderanlage 10, auf welcher Behälter 12 gefördert werden. Für die Förderanlage gilt das vorstehend Gesagte. Ebenfalls ersichtlich ist der Schnüffler 13, welcher über die Leitung 17 mit der Analyseeinrichtung 14 verbunden ist, für welche Bauteile ebenfalls die vorstehenden Erwägungen gelten. Die oberhalb der Behälter vorgesehenen Lufteinblasedüsen 18, 19 und 20 weisen jeweils einen unterschiedlichen Abstand von der Gasentnahmeposition auf. Die Düsen werden von einer gemeinsamen Leitung 29 aus einer nicht dargestellten Druckluftquelle gespiesen. Die Düsen 18, 19 und 20 sind jeweils über ein Ventil 21, 22 und 23 mit der Luftdruckleitung koppelbar bzw. von dieser trennbar. Die Ventile werden über elektrische Steuerleitungen von einer Steuereinrichtung geschaltet, welche in der Figur 2 zusammen mit der Analyseeinrichtung 14 gezeichnet ist, welche aber natürlich auch eine separate Steuereinrichtung sein kann. Auf die dargestellte Weise kann je nach Geschwindigkeit der Fördereinrichtung eine der Düsen aktiviert werden, deren Abstand von der Gasentnahmeposition dem gewünschten Abstand entspricht. Es kann auch mehr als eine Düse gleichzeitig aktiviert sein, wenn sich dies als vorteilhaft erweist. Auch die mehreren Düsen können geregelt verschoben werden, wie dies oben für die eine Düse erläutert worden ist.

Bei beiden gezeigten Ausführungsformen kann auch über die Luftgeschwindigkeit die Zeitkonstante zwischen dem Einblasen der Luft und dem Ausstossen des Behälterinhaltes geändert werden. So kann bei kleiner Geschwindigkeit auch mit kleinerem Druck eingeblasen werden. Bei grösserer Geschwindigkeit der Behälter hingegen kann mit höherem Druck eingeblasen werden. Weiter kann ein impulsweises Einblasen oder ein stetiges Einblasen gewählt werden. Vorzugsweise wird bei kleiner Geschwindigkeit impulsweise geblasen, bei grosser Geschwindigkeit hingegen dauernd.

Anstelle der dargestellten einen Düse bei Figur 1 bzw. der drei dargestellten Düsen in Figur 2 kann natürlich auch eine andere Anzahl von Düsen z.B. 2 oder 4 Düsen gewählt werden. Auch die Düsenanordnung mit mehreren Düsen kann zusätzlich in ihrem Abstand von der Gasentnahmeposition einstellbar bzw. regelbar sein. Die Ermittlung des jeweils günstigsten Abstandes ist aufgrund der oben angegebenen Parameter für den Fachmann ohne weiteres möglich und kann ansonsten auch empirisch ermittelt werden.

## Patentansprüche

1. Verfahren zur Prüfung von Behältern, insbesonders von Mehrweg-Flaschen, auf das Vorhandensein von Verunreinigungen, bei welchem durch eine Prüfeinrichtung in die auf einer Förderanlage (1; 10) geförderten Behälter (2; 12) ein Gas oder Gasgemisch, insbesondere Luft, eingeblasen und aus den Behältern Gasproben entnommen und analysiert werden, dadurch gekennzeichnet, dass die Gaseinblasung erfolgt, bevor der jeweilige Behälter die Gasprobenentnahmeposition erreicht.

2. Verfahren nach Anspruch 1, dadurch gekennzeichnet, dass die Gaseinblasung in einem vorbestimmten und einstellbaren Abstand von der Gasprobenentnahme erfolgt.

3. Verfahren nach Anspruch 1 oder 2, dadurch gekennzeichnet, dass der Abstand zwischen Gaseinblasung und der Gasprobenentnahme in Abhängigkeit von der Fördergeschwindigkeit und/oder der Behältergrösse, insbesonders Behälterhöhe, wählbar ist.

4. Verfahren nach einem der Ansprüche 1 bis 3, dadurch gekennzeichnet, dass der Abstand zwischen Gaseinblasung und Gasentnahme im Betrieb verstellbar ist und durch eine Regelung oder Steuerung verstellt wird, insbesonders in Abhängigkeit von der Fördergeschwindigkeit.

5. Verfahren nach einem der Ansprüche 1 bis 4, dadurch gekennzeichnet, dass der Abstand zwischen Gaseinblasung und der Gasprobenentnahme in Abhängigkeit vom Druck des einzublasenden Gases gewählt wird.

6. Verfahren nach einem der Ansprüche 1 bis 5, dadurch gekennzeichnet, dass die Gaseinblasung stetig oder impulsweise erfolgt.

7. Vorrichtung zur Prüfung von Behältern (2; 12), insbesonders Mehrweg-Flaschen, mit einer Prüfeinrichtung (1; 10) für die auf einer Förderanlage geförderten Behälter, wobei die Prüfeinrichtung zur Einblasung eines Gases oder Gasgemisches, insbesondere Luft, in die Behälter und zur Entnahme einer Gasprobe aus den Behältern ausgestaltet ist, dadurch gekennzeichnet, dass mindestens ein Einblasmittel (5; 18, 19, 20) in Förderrichtung vor der Gasprobenentnahmestelle angeordnet ist, durch welches Gas in den Behälter einblasbar ist bevor dieser die Gasentnahmeposition erreicht.

8. Vorrichtung nach Anspruch 7, dadurch gekennzeichnet, dass das Einblasmittel mehrere Düsen (18, 19, 20) in verschiedenem Abstand von der Gasprobenentnahmestelle umfasst.

9. Vorrichtung nach Anspruch 8, dadurch gekennzeichnet, dass die Düsen mittels vorgeschalteter Ventile (21, 22, 23) einzeln mit Gas speisbar sind.

10. Vorrichtung nach einem der Ansprüche 7 bis 9, dadurch gekennzeichnet, dass das Einblasmittel stetig oder impulsweise betätigbar ist.

11. Vorrichtung nach einem der Ansprüche 7 bis 10, dadurch gekennzeichnet, dass das Einblasmittel durch einen Antrieb in seinem Abstand von der Gasprobenentnahmestelle einstellbar ist, und dass ein Steuermittel oder Regelungsmittel vorgesehen ist, welches den Abstand bestimmt.

12. Vorrichtung nach einem der Ansprüche 7 bis 11, dadurch gekennzeichnet, dass die Förderanlage im Bereich der Vorrichtung linear verläuft.
